(19) Europäisches Patentamt
European Patent Office
Office européen des brevets

(11) Numéro de publication: **0 240 391 B1**

(12) **FASCICULE DE BREVET EUROPEEN**

(45) Date de publication de fascicule du brevet: **10.07.91**

(51) Int. Cl.[5]: **C07D 471/04**, A61K 31/495, //(C07D471/04,221:00,221:00)

(21) Numéro de dépôt: **87400487.2**

(22) Date de dépôt: **05.03.87**

Le dossier contient des informations techniques présentées postérieurement au dépôt de la demande et ne figurant pas dans le présent fascicule.

(54) **Sel d'argent dérivé de naphtyridine, utile notamment comme antibactérien, procédé de préparation et compositions pharmaceutiques le contenant.**

(30) Priorité: **05.03.86 FR 8603092**

(43) Date de publication de la demande: **07.10.87 Bulletin 87/41**

(45) Mention de la délivrance du brevet: **10.07.91 Bulletin 91/28**

(84) Etats contractants désignés: **AT BE CH DE ES FR GB IT LI NL**

(56) Documents cités: **EP-A- 0 049 593**

**Il Farmaco - Ed.Sc., vol. 39, page 910 (1984)**

(73) Titulaire: **Edmond Pharma Srl**
**Via dei Giovi 131**
**I-20037 Paderno Dugnano (Milano)(IT)**

(72) Inventeur: **Gobetti, Marino**
**Via Valdagno 7**
**Milano(IT)**
Inventeur: **Vandoni, Guido**
**Via Principale 30**
**Correzzana (Milano)(IT)**

(74) Mandataire: **Gillard, Marie-Louise et al**
**Cabinet Beau de Loménie 55, Rue d'Amsterdam**
**F-75008 Paris(FR)**

**EP 0 240 391 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un nouveau produit ayant une remarquable activité antibactérienne et antifongique par la voie topique, ainsi que l'utilisation de ce produit pour la préparation de compositions pharmaceutiques pour le traitement des états infectieux de la peau et les compositions pharmaceutiques ainsi obtenues. Elle concerne également un procédé de préparation dudit produit.

Plus particulièrement, la présente invention concerne le sel d'argent de l'acide 1-éthyl-6-fluoro-1,4-dihydro-4-oxo-7-(1-pipérazinyl)-1,8-naphtyridine-3-carboxylique de formule

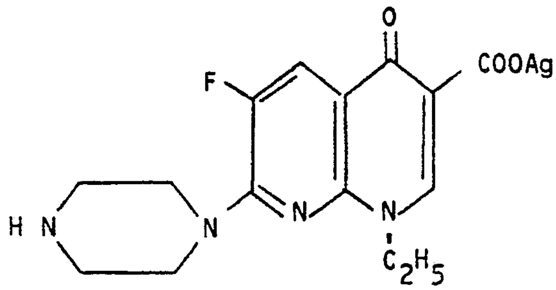

I

ainsi que des compositions pharmaceutiques contenant le composé I ci-dessus comme ingrédient actif.

Le brevet européen 9425 décrit l'acide 1-éthyl-6-fluoro-1,4-dihydro-4-oxo-7-(1-pipérazinyl)-1,8-naphtyridine-3-carboxylique, à savoir l'acide libre correspondant au composé de formule I ci-dessus.

Le même brevet cite également les sels de cet acide acceptables en pharmacie, mais il ne décrit spécifiquement que les sels d'addition d'acides, notamment le chlorhydrate, l'acétate et le méthanesulfonate.

Le brevet européen 49593 décrit, entre autres, le sel d'argent de l'acide 1-éthyl-6-fluoro-1,4-dihydro-4-oxo-7-(1-pipérazinyl)-quinolinyl-3-carboxilique et son activité antibactérienne qui est du même ordre que celle de l'acide libre correspondant.

La publication M. Artico et al., Il Farmaco - Ed. Sci. 1984, 39, 910-924, décrit l'acide l-éthyl-1,4-dihydro-4-oxo-7-(l-pyrryl) quinoline-3-carboxylique (piroxacine) et certains de ses dérivés, à activité anti-microbienne. L'activité anti-fongique de ce produit est faible, quoique supérieure à celle des produits de référence, à savoir l'acide nalidixique, l'acide pipémidique, l'acide piromidique et le Nax 9 (acide l-éthyl-1,4-dihydro-4-oxo-7-(1-pyrrolidinyl)quinoline-3-carboxylique).

On a maintenant trouvé que le sel d'argent de formule I ci-dessus possède une activité antibactérienne très élevée lorsqu'il est placé directement sur le lieu de l'infection, notamment en cas de brûlures.

On a également trouvé que le composé de formule I ci-dessus est plus actif que l'acide libre correspondant.

On a encore trouvé d'une façon surprenante que le composé de formule I ci-dessus, bien qu'il soit un sel d'argent, est moins photosensible que d'autres sels d'argent antibactériens, par exemple le sel d'argent de la sulfadiazine, non seulement à l'état solide, mais aussi en solution.

On a enfin trouvé que le sel d'argent de formule I possède non seulement une excellente activité antibactérienne mais également une activité antifongique, notamment sur des champignons des espèces Candida et Trichophyton. Bien que l'activité antifongique soit inférieur en termes de Concentration Minimale d'Inhibition (CMI), à l'activité antibactérienne, l'utilisation à laquelle le composé de la présente invention est destiné, à savoir le traitement des infections par voie locale, comporte l'application directe du principe actif sur le lieu de l'infection. Par conséquent, ledit principe actif agit directement sur les champignons à des concentrations largement suffisantes.

Ainsi, la présente invention concerne, en tant que produit nouveau, le sel d'argent de l'acide 1-éthyl-6-fluoro-1,4-dihydro-4-oxo-7-(1-pipérazinyl)-1,8-naphtyridine-3-carboxylique de formule I.

Le composé de la présente invention est utile comme médicament dans le traitement local des infections et, notamment, des brûlures chez l'homme et les animaux.

La présente invention concerne en même temps un procédé pour la préparation du sel d'argent de l'acide 1-éthyl-6-fluoro-1,4-dihydro-4-oxo-7-(1-pipérazinyl)-1,8-naphtyridine-3-carboxylique, caractérisé en ce qu'on dissout l'acide libre correspondant dans une solution aqueuse d'un hydroxyde alcalin et on traite la solution aqueuse du sel alcalin ainsi obtenue avec une solution aqueuse d'un sel d'argent.

Il est préférable que l'acide de départ soit utilisé en faible excès et que l'acide non salifié soit éliminé par filtration.

Comme hydroxyde alcalin, on utilise de préférence l'hydroxyde de sodium ou de potassium; le sel d'argent préféré est le nitrate d'argent. Par addition de la solution aqueuse du sel d'argent à la solution aqueuse du sel alcalin, le composé de formule I précipite et peut être isolé par filtration.

Il est souhaitable que le produit final soit traité comme un produit photosensible, bien que sa photosensibilité soit inférieure à celle d'autres sels d'argent antibactériens. Ainsi, les opérations finales, surtout le séchage, devraient être effectuées à l'abri de la lumière.

Pour l'évaluation des activités antibactérienne et antifongique ont été testées 24 souches de bactéries et 11 de champignons isolées de matériel pathologique humain récent ou provenant de collections internationales, ainsi subdivisées :

| Souches | Nombre |
|---|---|
| Bactéries gram-positives | |
| - Staphylococcus aureus (dont 2 penicillino-résistantes) | 6 |
| - Staphylococcus epidermidis | 3 |
| - Staphylococcus pyogenes (béta-hémolytique de groupe A) | 4 |
| - Streptococcus faecalis | 2 |

| Bactéries gram-négatives | Nombre |
|---|---|
| - Escherichia coli | 5 |
| - Pseudomonas aeruginosa | 4 |
| Mycetes | |
| - Candida albicans | 5 |
| - Trichophyton mentographytes | 4 |
| - Trichophyton rubrum | 2 |

Pour l'activité in vitro on a déterminé la CMI par la méthode des dilutions (milieu agar) selon la technique classique.

La solution mère des produits à tester (composé de formule I et acide libre de départ) a été préparée par dissolution dans le diméthylsulfoxyde et addition d'eau distillée dans le rapport 1 : 10. Les solutions ultérieures ont été préparées par dilution avec de l'eau distillée ou avec le terrain minimum essential d'Eagle contenant 3 % de serum de veau.

Les concentrations ont été exprimées en µg de substance active, calculée comme acide libre, par ml de milieu.

Le tableau I montre l'activité antibactérienne in vitro du sel d'argent de formule I ci-dessus (Composé I) et de l'acide libre correspondant (Acide libre), exprimée par le nombre de souches sensibles aux produits testés à des concentrations de 30 à 0,01 µg/ml.

TABLEAU I

| Souches | n. | Nombre de souches sensibles aux concentrations suivantes (µg/ml) | | | | | | | |
|---|---|---|---|---|---|---|---|---|---|
| | | 30 | 10 | 3 | 1 | 0,3 | 0,1 | 0,03 | 0,01 |
| Staphylococcus aureus | 4 | | | | | | | | |
| - Composé I | | | | | | 1 | 3 | | |
| - Acide libre | | | | | 2 | | 1 | | |
| Staphylococcus aureus (pen rés.) | 2 | | | | | | | | |
| - Composé I | | | | | | 1 | 1 | | |
| - Acide libre | | | | | 1 | 1 | | | |
| Streptococcus epidermidis | 3 | | | | | | | | |
| - Composé I | | | | 1 | 1 | 1 | | | |
| - Acide libre | | | 1 | 2 | | | | | |
| Streptococcus pyogenes | 4 | | | | | | | | |
| - Composé I | | | 1 | 2 | 1 | | | | |
| - Acide libre | | | 2 | 1 | 1 | | | | |
| Streptococcus faecalis | | | | | | | | | |
| - Composé I | | | | 1 | 1 | | | | |
| - Acide libre | | 1 | 1 | | | | | | |
| Escherichia coli | | | | | | | | | |
| - Composé I | | | | | | 2 | 2 | 1 | |
| - Acide libre | | | | | | 2 | 2 | 1 | |
| Pseudomonas aeruginosa | | | | | | | | | |
| - Composé I | | | | | | | 3 | 1 | |
| - Acide libre | | | | | 1 | 3 | | | |

Les résultats résumés dans le tableau I montrent que la transformation de l'acide libre en son sel d'argent a influencé de façon positive l'activité sur les bactéries gram-positives et gram-négatives. En effet, le rapport d'activité entre les deux est en général en faveur du sel d'argent de formule I. Notamment, la salification augmente de façon décisive l'activité sur les souches de Pseudomonas aeruginosa.

Le tableau II montre l'activité antifongique du sel d'argent de formule I ci-dessus, exprimée par le nombre de souches sensibles à des concentrations de 3O à 1µ g/ml.

TABLEAU II

| Souche | n. | nombre de souches sensibles aux concentrations suivantes (en µg/ml) | | | |
|---|---|---|---|---|---|
| | | 30 | 10 | 3 | 1 |
| Candida albicans | 5 | 1 | 3 | 1 | |
| Trichophyton mentagrophytes | 4 | 1 | 2 | 1 | |
| Trichophyton rubrum | 2 | 1 | 1 | | |

Les résultats résumés dans le tableau II montrent que le sel d'argent de formule I est doté d'une activité antifongique indiscutable. L'acide libre, qui a été essayé sur les mêmes souches s'est montré inactif jusqu'à des concentrations de 100µ g/ml.

Le composé de formule I ci-dessus peut donc être utilisé comme substance thérapeutiquement active, notamment comme agent antibactérien et antifongique par voie locale.

Bien que le composé de la présente invention puisse, en cas de nécessité, être appliqué tel quel sur le site de l'infection, il sera de préférence mélangé à des excipients pharmaceutiques adaptés à l'administration topique.

Ainsi, la présente invention a également pour objet l'utilisation du composé de formule I comme produit antibactérien et antifongique et la préparation de compositions pharmaceutiques pour l'application locale.

La présente invention concerne enfin des compositions pharmaceutiques contenant, comme ingrédient actif, le composé de formule I ci-dessus, seul ou en mélange avec un excipient pharmaceutique.

Les compositions de la présente invention contiennent l'ingrédient actif, éventuellement micronisé, en mélange intime avec l'excipient et peuvent se présenter sous forme solide, semi-solide, liquide ou visqueuse. L'ingrédient actif peut être formulé avec les excipients usuels pour poudres, onguents, lotions, crèmes, émulsions ou suspensions aqueuses ou d'autre forme d'usage topique.

Les excipients sont ceux qui sont couramment utilisés dans la préparation de formulations à usage topique comme, par exemple, les graisses d'origine animale ou végétale, les huiles végétales, les acides gras saturés ou insaturés, les alcools, les polyalcools tels que le glycérol, le propylèneglycol ou les polyéthylèneglycols, les esters des sucres tels que le monostéarate de saccharose, le monooléate de saccharose ou les polyesters de saccharose, les cires, les hydrocarbures aliphatiques de haut poids moléculaire ou la lanoline, éventuellement en présence d'eau. D'autres excipients qui peuvent être utilisés sont les bases hydrophiles, le cholestérol, la vaseline, l'huile de vaseline, les silicones physiologiquement inertes et les alginates.

Les compositions de la présente invention peuvent également contenir des agents de conservation, comme le p-hydroxybenzoate de méthyle ou de propyle ou des émulsifiants.

Le sel d'argent de l'acide 1-éthyl-6-fluoro-1,4-dihydro-4-oxo-7-(1-pipérazinyl)-1,8-naphtyridine-3-carboxylique qui constitue le principe actif est contenu dans les compositions pharmaceutiques de la présente invention en quantité suffisante pour produire l'effet thérapeutique souhaité sur le site de l'infection ou sur la brûlure.

Des compositions convenables contiennent de 0,001 à 5 % en poids d'ingrédient actif, de préférence de 0,01 à 1 %.

Les compositions pharmaceutiques de la présente invention sont appliquées localement une ou plusieurs fois par jour sur la surface des infections ou des brûlures chez l'homme ou l'animal.

Les exemples suivants illustrent l'invention.

Exemple 1

On dissout 0,11 mol d'acide 1-éthyl-6-fluoro-1,4-dihydro-4-oxo-7-(1-pipérazinyl)-1,8-naphtyridine-3-carboxylique dans 100 ml (0,1 mol) de solution d'hydroxyde de sodium N, puis on filtre l'excès d'acide qui n'a pas réagi. A la solution limpide ainsi obtenue, on ajoute une solution de 0,1 mol de nitrate d'argent dans 200 ml d'eau.

On filtre le précipité qui s'est formé, on le lave à fond avec de l'eau et on le sèche sous pression réduite à 50° C à l'abri de la lumière. On obtient ainsi le sel d'argent de l'acide 1-éthyl-6-fluoro-1,4-dihydro-4-oxo(7-(1-pipérazinyl)-1,8-naphtyridine-3-carboxylique, F. 178° C (déc). Rendement : 98% de la théorie.

Exemple 2

Crème contenant comme ingrédient actif le composé de l'exemple 1 et ayant la composition suivante :

| Composants | % en poids |
|---|---|
| Ingrédient actif | 0,3 |
| Cholestérol pur | 3,0 |
| Alcool stéarylique | 8,0 |
| Paraffine blanche molle | 51,0 |
| Paraffine liquide | 37,7 |

L'ingrédient actif est ajouté aux autres composants préalablement fondus à 75° C et le mélange est agité jusqu'à solidification.

Exemple 3

On prépare comme décrit à l'exemple 2 une crème contenant comme ingrédient actif le composé de l'exemple 1 et ayant la composition suivante :

| Composants | % en poids |
|---|---|
| Ingrédient actif | 0,20 |
| Lanoline | 14,45 |
| Paraffine liquide | 17,75 |
| Paraffine blanche molle | 67,60 |

Exemple 4

Crème hydrophile contenant comme ingrédient actif le composé de l'exemple 1 et ayant la composition suivante :

| Composants | % en poids |
|---|---|
| Ingrédient actif | 0,300 |
| p-hydroxybenzoate de propyle | 0,015 |
| p-hydroxybenzoate de méthyle | 0,025 |
| Stéarate de Polyoxyl 40 | 5,000 |
| Propylèneglycol | 12,000 |
| Alcool stéarylique | 25,000 |
| Paraffine blanche molle | 25,000 |
| Eau distillée | q.s.p. 100,000 |

On fait fondre l'alcool stéarylique et la paraffine blanche molle au bain de vapeur en chauffant à environ 75 °C, on ajoute à la masse ainsi obtenue l'ingrédient actif dans le propylèneglycol, puis les autres ingrédients, préalablement dissous dans l'eau et chauffés à 75 °C. On agite ensuite le mélange jusqu'à solidification.

Exemple 5

Crème contenant comme ingrédient actif le composé de l'exemple 1 et ayant la composition suivante :

| Composants | % en poids |
|---|---|
| Ingrédient actif | 0,1 |
| Alcool cétylique | 0,5 |
| Lanoline anhydre | 5,0 |
| Eau distillée | 5,0 |
| Paraffine liquide | 20,0 |
| Paraffine blanche molle | 69,4 |

On fait fondre l'alcool cétylique, la paraffine liquide et la paraffine blanche molle à 75 °C, on ajoute à la masse fondue l'ingrédient actif, puis la lanoline préalablement mélangée à l'eau. La masse est ensuite raffinée deux fois.

Exemple 6

Crème contenant comme ingrédient actif le composé de l'exemple 1 et ayant la composition suivante :

| Composants | % en poids |
|---|---|
| Ingrédient actif | 1,0 |
| Alcool cétostéarylique | 12,0 |
| Paraffine blanche molle | 6,5 |
| Paraffine liquide | 6,5 |
| Stéarate d'isopropyle | 3,0 |
| Propylèneglycol | 3,0 |
| p-hydroxybenzoate de méthyle | 0,2 |
| p-hydroxybenzoate de propyle | 0,1 |
| "Tween 80" (marque déposée) | 0,2 |
| Polyéthylèneglycol 6000 | 4,2 |
| Eau distillée | 63,3 |

On fait fondre l'alcool cétostéarylique, la paraffine blanche molle et le stéarate d'isopropyle à 70° C, on ajoute à la masse ainsi obtenue l'ingrédient actif dans le propylèneglycol, puis les autres composants préalablement mélangés à l'eau et chauffés à 70° C. La masse est ensuite raffinée deux fois.

Il est entendu que l'invention n'est pas limitée aux modes de réalisation préférés décrits ci-dessus à titre d'illustration et que l'homme de l'art pourra y apporter des modifications sans sortir du cadre de l'invention.

**Revendications**

**Revendications pour les Etats contractants suivants: BE CH DE FR GB IT LI NL**

1. Sel d'argent dérivé de naphtyridine, caractérisé en ce qu'il consiste en le sel d'argent de l'acide 1-éthyl-6-fluoro-1,4-dihydro-4-oxo-7(1-pipérazinyl)-1,8-naphtyridine-3-carboxylique de formule

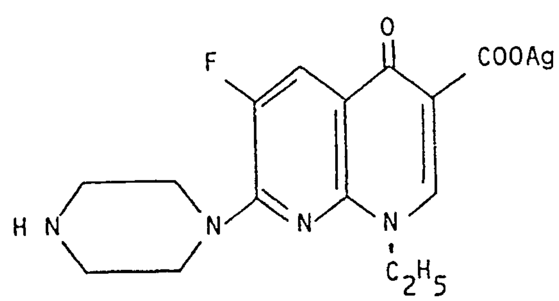

I

2. Procédé pour la préparation du composé selon la revendication 1, caractérisé en ce qu'on dissout l'acide 1-éthyl-6-fluoro-1,4-dihydro-4-oxo-7-(1-pipérazinyl)-1,8-naphtyridine-3-carboxylique dans une solution aqueuse d'un hydroxyde alcalin et on traite la solution aqueuse du sel alcalin ainsi obtenue avec une solution aqueuse d'un sel d'argent.

3. Procédé selon la revendication 2, caractérisé en ce que l'on utilise comme hydroxyde alcalin, l'hydroxyde de sodium ou de potassium.

4. Procédé selon l'une des revendications 2 ou 3, caractérisé en ce que l'on utilise comme sel d'argent, le nitrate.

5. Compositions pharmaceutiques notamment antibactériennes et antifongiques, caractérisées en ce qu'elles contiennent , comme ingrédient actif, le composé selon la revendication 1.

6. Compositions pharmaceutiques antibactériennes et antifongiques selon la revendication 5, pour l'application locale, caractérisées en ce qu'elles contiennent, comme ingrédient actif, le composé de la revendication 1 en mélange avec un excipient pharmaceutique.

7. Compositions pharmaceutiques selon la revendication 6, caractérisées en ce qu'elles contiennent de 0,001 à 5 % d'ingrédient actif.

8. Compositions pharmaceutiques selon la revendication 6, caractérisées en ce qu'elles contiennent de 0,01 à 1 % d'ingrédient actif.

**Revendications pour les Etats contractants suivants: AT ES**

1. Procédé pour la préparation du sel d'argent de l'acide 1-éthyl-6-fluoro-1,4-dihydro-4-oxo-7-(1-pipérazinyl)-1,8-naphtyridine -3-carboxylique de formule

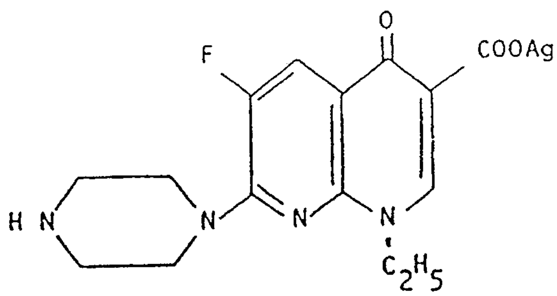

. I

caractérisé en ce qu'on dissout l'acide 1-éthyl-6-fluoro-1,4-dihydro-4-oxo-7-(1-pipérazinyl)-1,8-naphtyridine-3-carboxylique dans une solution aqueuse d'un hydroxyde alcalin et on traite la solution aqueuse du sel alcalin ainsi obtenue avec une solution aqueuse d'un sel d'argent.

2. Procédé selon la revendication 1, caractérisé en ce que l'on utilise comme hydroxyde alcalin, l'hydroxyde de sodium ou de potassium.

3. Procédé selon l'une des revendications 1 ou 2, caractérisé en ce que l'on utilise comme sel d'argent, le nitrate.

**Claims**

**Claims for the following Contracting States: DE, GB, FR, IT, NL, CH/LI, BE**

1. Silver salt derivative of naphthyridine, characterized in that it consists in the silver salt of 1-ethyl-6-fluoro-1,4-dihydro-4-oxo-7-(piperazin-1-yl)-1,8-naphthyridine-3-carboxylic acid of formula

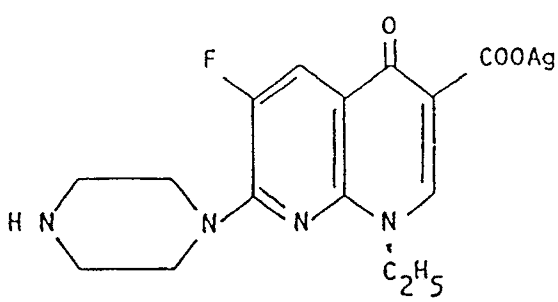

. I

2. A process for the preparation of a compound according to claim 1, characterized in that the 1-ethyl-6-fluoro-1,4-dihydro-4 oxo-7-(piperazin-1-yl)-1,8-naphthyridine-3-carboxylic acid is dissolved in an aqueous solution of an alcaline hydroxide, and that the so-obtained solution of alcaline salt is treated with an aqueous solution of a silver salt.

3. A process according to claim 2, characterized in that sodium hydroxide or potassium hydroxide is used as alcaline hydroxide.

4. A process according to claims 2 or 3, characterized in that the nitrate is used as silver salt.

5. Pharmaceutical compositions, particularly anti-bacterial and anti-fungus compositions, characterized in that they contain, as active ingredient, the compound according to claim 1.

6. Anti-bacterial and anti-fungus pharmaceutical compositions according to claim 5, for topical application, characterized in that they contain, as active ingredient, the compound of claim 1 in admixture with a pharmaceutical vehicle.

7. Pharmaceutical compositions according to claim 6, characterized in that they contain 0,001 to 5 % of active ingredient.

8. Pharmaceutical compositions according to claim 6, characterized in that they contain 0,01 to 1 % of active ingredient.

**Claims for the following Contracting States: AT, ES**

1. A process for the preparation of the silver salt of 1-ethyl-6-fluoro-1,4-dihydro-4-oxo-7-(piperazin-1-yl)-1,8-naphthyridine-3-carboxylic acid of formula

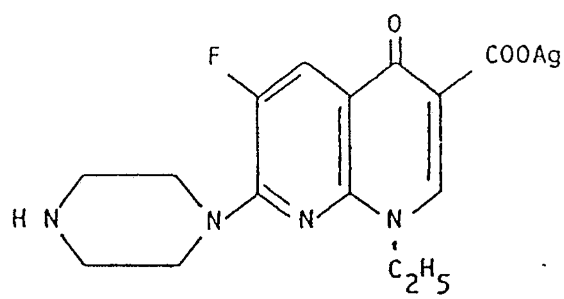

I

characterized in that the 1-ethyl-6-fluoro-1,-4-dihydro-4-oxo-7-(piperazin-1-yl)-1,8-naphthyridine-3-carboxylic acid is dissolved in an aqueous solution of an alcaline hydroxide, and that the so-obtained solution of alcaline salt is treated with an aqueous solution of a silver salt.

2. A process according to claim 2, characterized in that sodium hydroxide or potassium hydroxide is used as alcaline hydroxide.

3. A process according to claims 2 or 3, characterized in that the nitrate is used as silver salt.

**Ansprüche**

**Patentansprüche für folgende Vertragsstaaten: DE, GB, FR, IT, NL, CH/LI, BE**

1. Von Naphthyridin abgeleitetes Silbersalz, dadurch gekennzeichnet, daß es aus dem Silbersalz der 1-Ethyl-6-fluor-1,4-dihydro-4-oxo-7-(1-piperazinyl)-1,8-naphthyridin-3-carbonsäure der Formel

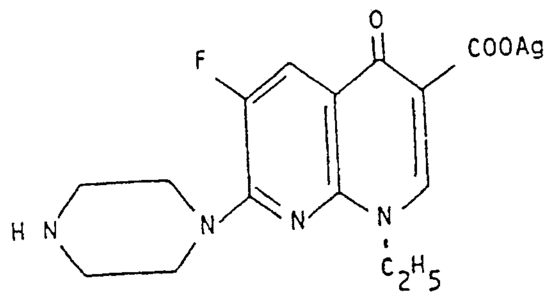

I

besteht.

2. Verfahren zur Herstellung der Verbindung nach Anspruch 1, dadurch gekennzeichnet, daß man 1-Ethyl-6-fluor-1,4-dihydro-4-oxo-7-(1-piperazinyl)-1,8-naphthyridin-3-carbonsäure in einer wässerigen Lösung eines Alkalihyroxids löst und die wässerige Lösung des so erhaltenen Alkalisalzes mit einer wässerigen Lösung eines Silbersalzes behandelt.

3. Verfahren nach Anspruch 2, dadurch gekennzeichnet, daß man als Alkalihydroxid Natriumhydroxid oder Kaliumhydroxid verwendet.

4. Verfahren nach einem der Ansprüche 2 oder 3, dadurch gekennzeichnet, daß man als Silbersalz das Nitrat verwendet.

5. Insbesondere antibakterielle und antifungizide pharmazeutische Zusammensetzungen, dadurch gekennzeichnet, daß sie als aktives Ingrediens die Verbindung nach Anspruch 1 enthalten.

6. Antibakterielle und antifugizide pharmazeutische Zusammensetzungen nach Anspruch 5 für lokale Anwendung, dadurch gekennzeichnet, daß sie als aktives Ingrediens die Verbindung des Anspruchs 1 in Mischung mit einem pharmazeutischen Exzipienten enthalten.

7. Pharmazeutische Zusammensetzungen nach Anspruch 6, dadurch gekennzeichnet, daß sie 0,001 bis 5 % aktives Ingrediens enthalten.

8. Pharmazeutische Zusammensetzungen nach Anspruch 6, dadurch gekennzeichnet, daß sie 0,01 bis 1 % aktives Ingrediens enthalten.

**Patentansprüche für folgende Vertragsstaaten: AT, ES**

1. Verfahren zur Herstellung des Silbersalzes der 1-Ethyl-6-fluor-1,4-dihydro-4-oxo-7-(1-piperazinyl)-1, 8-naphthyridin-3-carbonsäure der Formel

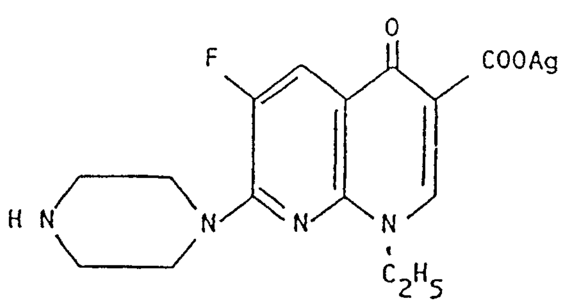

I

dadurch gekennzeichnet, daß man 1-Ethyl-6-fluor-1,4-dihydro-4-oxo-(1-piperazinyl)-1,8-naphthyridin-3-carbonsaüre in einer wässerigen Lösung eines Alkalihyroxidslöst und die wässerige Lösung des so erhaltenen Alkalisalzes mit einer wässerigen Lösung eines Silbersalzes behandelt.

2. Verfahren nach Anspruch 1, dadurch gekennzeichnet, daß man als Alkalihydroxid Natriumhydroxid oder

Kalium hydroxid verwendet.

3. Verfahren nach einem der Ansprüche 1 oder 2, dadurch gekennzeichnet, daß man als Silbersalz das Nitrat verwendet.